# NOUVEAU FASCICULE DE BREVET EUROPEEN

(11) **EP 0 575 604 B2**
(45) Date de publication et mention de la décision concernant l'opposition: **26.03.2003**
(45) Mention de la délivrance du brevet: 06.12.1995
(21) Numéro de dépôt: 93904076.2
(22) Date de dépôt: 13.01.1993
(51) Int. Cl.: A61K 7/00, A61K 7/13, C08G 61/12, C09B 69/10

(54) **PRODUITS INDOLINIQUES, LEURS PROCEDES DE PREPARATION ET LEUR UTILISATION EN COSMETIQUE**
INDOLIN-PRODUKTE, IHRE VERFAHREN ZU DEREN HERSTELLUNG SOWIE IHRE VERWENDUNG IN DER KOSMETIK
INDOLINE PRODUCTS, METHODS FOR PREPARING SAME, AND USE THEREOF IN COSMETICS

(30) Priorité: 16.01.1992 FR 9200416
(43) Date de publication de la demande: 29.12.1993
(73) Titulaire: L'OREAL, 75008 Paris (FR)
(72) Inventeur: LAGRANGE, Alain, F-78400 Chatou (FR); ANDREAN, Hervé, F-75014 Paris (FR); JUNINO, Alex, F-93190 Livry-Gargan (FR)
(74) Mandataire: Casalonga, Axel
(86) Numéro de dépôt international: FR9300030
(87) Numéro de publication internationale: WO93013744

(56) Documents cités:
- EP-A- 0 379 409
- EP-A- 0 441 689
- EP-A- 0 462 857
- WO-A-90/01919
- WO-A-90/10430
- WO-A-91/17739
- DE-A- 3 824 094
- DE-C- 1 916 139
- GB-A- 2 207 153
- US-A- 4 013 404

## Description

La présente invention est relative à des produits indoliniques, à leurs procédés de préparation, leur utilisation en cosmétique et aux compositions cosmétiques les mettant en oeuvre.

L'utilisation de pigments colorés présente un très grand intérêt dans le domaine cosmétique, notamment dans les produits de maquillage destinés au maquillage des phanères et/ou de la peau.

On utilise généralement des pigments minéraux ou des pigments issus de colorants directs de synthèse ou de carbone pur dans le cas des pigments noirs. Ces différents produits présentent suivant les applications, des problèmes de mise en oeuvre et ne sont pas toujours exempts de problèmes au niveau compatibilité et toxicologie.

La demanderesse a découvert de nouveaux produits utilisables comme pigments, en particulier en cosmétique qui sont des produits indoliniques, particulièrement intéressants au niveau des colorations qu'ils permettent d'obtenir ainsi que dans leur utilisation cosmétique.

Les produits conformes à l'invention sont obtenus par un processus de polymérisation oxydative mettant en oeuvre au moins une indoline.

Par analogie et simplification, on appellera "produit indolinique" ou "polymère indolinique, le produit obtenu par polymérisation oxydative de différents composés comportant au moins une indoline.

La présente invention a donc pour objet de nouveaux produits indoliniques tels que définis ci-après.

Un autre objet de l'invention est constitué par le procédé de préparation de ces produits.

L'invention a également pour objet l'application cosmétique de ces produits indoliniques, notamment les produits de maquillage de la peau et/ou des phanères et de la protection de l'épiderme humain contre les rayonnements UV.

D'autres objets de l'invention apparaîtront à la lecture de la description et des exemples qui suivent.

Les produits indoliniques conformes à l'invention sont des produits isolés sous forme de particules ayant une granulométrie moyenne inférieure à 50 µm et qui résultent de la polymérisation oxydative d'un ou plusieurs composés indoliniques répondant à la formule : 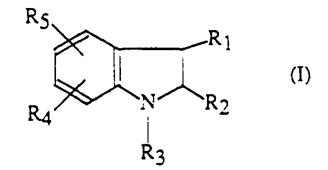 dans laquelle :
R₁ et R₃ représentent, indépendamment l'un de l'autre, un atome d'hydrogène ou un groupement alkyle en C₁-C₄;
R₂ représente un atome d'hydrogène, un groupement alkyle en C₁-C₄, un groupement carboxyle ou alcoxy(C₁-C₄)carbonyle;
R₄ désigne un atome d'hydrogène, un groupement alkyle en C₁-C₄, hydroxyle, alcoxy(C₁-C₄), amino ou alkylamino en C₁-C₁₀ ou halogène;
R₅ désigne un atome d'hydrogène, un groupement hydroxyle, alcoxy en C₁-C₄ ou amino;
   avec la condition qu'au moins un des radicaux R₄ ou R₅ désigne un groupement hydroxyle, alcoxy ou amino; et sous réserve que lorsque R₅ désigne un groupement amino, R₄ ne peut désigner un radical alkylamino;
R₄ et R₅ peuvent également former un cycle alkylènedioxy en C₁-C₂, et sont en position 5 et 6;
ainsi que les sels correspondants.

Parmi les composés répondant à la formule (I), les composés préférés utilisés conformément à l'invention, sont choisis parmi la 5,6-dihydroxyindoline, la 6-hydroxyindoline, la 5,6-méthylènedioxyindoline, la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6-aminoindoline, la 5,6-diaminoindoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amino 6-chloroindoline, la 4-amino 6-iodoindoline, la 4-amino 5-bromoindoline, la 4-amino 5-hydroxyindoline, la 4-amino 7-hydroxyindoline, la 4-amino 5-méthoxyindoline, la 4-amino 7-méthoxyindoline, la 5-aminoindoline, la 2,3-diméthyl 5-aminoindoline, la 1-méthyl 5-aminoindoline, la 2-méthyl 5-aminoindoline, la 5-N-(1-méthylhexyl)aminoindoline, la 5,6-diméthoxyindoline et la 5,6-dihydroxy 2-carboxyindoline.

Dans les composés de formule (I), les radicaux alkyle en C₁-C₄ désigent de préférence méthyle, éthyle, propyle, isopropyle, butyle, isobutyle. Pour les radicaux alkyle en C₁-C₁₀, le radical alkyle en C₁-C₁₀ désigne de préférence méthyle, éthyle, propyle, isopropyle, butyle, isobutyle, 1-méthylhexyle, 1-méthylheptyle, 1-méthyloctyle; les radicaux alcoxy désignent de préférence méthoxy, éthoxy, propoxy et butoxy; halogène désigne brome, chlore ou iode.

Les sels sont des sels cosmétiquement acceptables, en particulier des chlorhydrates, bromhydrates, sulfates, méthanesulfonates. Les bromhydrates des composés ci-dessus sont particulièrement préférés.

Les produits indoliniques conformes à l'invention peuvent être obtenus par cooxydation d'au moins une indoline répondant à la formule (I) ci-dessus, avec au moins un dérivé d'indole choisi parmi les mono-, les dihydroxyindoles ou les aminoindoles décrits plus particuliérement dans le brevet EP-A-239 826 et les demandes de brevet EP-A-425 345 et GB-A-2 224 754, dans des proportions de jusqu'à 50% en moles de dérivés d'indole par rapport au nombre total de moles de composés à oxyder.

Les dérivés d'indole sont choisis plus particulièrement parmi les composés de formule : 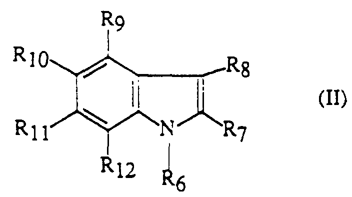 dans laquelle :
R₆ et R₈ désignent, indépendamment l'un de l'autre, un atome d'hydrogène ou un groupe alkyle en C₁-C₄;
R₇ représente un atome d'hydrogène, un groupement alkyle en C₁-C₄, un groupement carboxyle ou un groupement alcoxy(C₁-C₄) carbonyle;
R₉ et R₁₂ désignent, indépendamment l'un de l'autre, un atome d'hydrogène, un groupement hydroxy, un groupe alkyle en C₁-C₄,amino, alcoxy(C₁-C₄), acyl(C₂-C₄)oxy, acyl(C₂-C₄)amino;
R₁₀ désigne hydrogène, un groupement hydroxy, alcoxy(C₁-C₄), alkyle(C₁-C₄), halogène, amino, acyl-(C₂-C₁₄)oxy, acyl(C₂-C₄)amino, triméthylsilyloxy;
R₁₁ désigne hydrogène, un groupement hydroxy, alcoxy(C₁-C₄), amino, acyl(C₂-C₄)oxy, acyl(C₂-C₄)-amino, triméthylsilyloxy hydroxyalkyl(C₂-C₄)amino;
R₁₀ et R₁₁ peuvent former, conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy;
au moins l'un des groupements R₉ à R₁₂ représente un groupement OZ ou NHR, un seul au plus des groupements R₉ à R₁₂ désignant NHR; et au plus deux des groupements R₉ à R₁₂ désignent OZ, dans le cas où Z désigne hydrogène, ces groupements sont en position 5 et 6; et au moins un des groupements R₉ à R₁₂ représente hydrogène, dans le cas où un seul de ces groupements désigne hydrogène, un seul groupement parmi R₉ à R₁₂ désigne alors NHR ou OZ, et les autres groupements désignent alkyle en C₁-C₄;
R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄, triméthylsilyle;
et les sels correspondants.

Les composés indoliques de formule (II) sont choisis, en particulier, parmi le 4-hydroxyindole, 5-hydroxyindole, 6-hydroxyindole, 7-hydroxyindole, le 4-hydroxy 5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, 5-aminoindole, 6-aminoindole, 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole.

Le 5,6-dihydroxyindole, le 6-hydroxyindole, le 3-méthyl 5,6-dihydroxyindole, le 2-méthyl 5,6-dihydroxyindole, le 1-méthyl 5,6-dihydroxyindole, le 2-carboxy 5,6-dihydroxyindole, utilisés seuls ou en mélange, sont préférés.

Les produits indoliniques conformes à l'invention, peuvent être préparés selon différents processus de polymérisation oxydative.

Selon un premier type de processus, on procède à une simple oxydation à l'air. Dans ce cas, on n'utilise pas d'autre agent oxydant que l'oxygène de l'air et on opère de préférence à un pH alcalin dans un milieu eau ou eau/solvant.

L'oxydation à l'air peut également s'effectuer en présence d'un agent alcalin et/ou d'un catalyseur métallique d'oxydation tel que l'ion cuivrique.

Selon un deuxième type de procédé, la préparation des produits indoliniques conformes à l'invention peut s'effectuer en présence d'un agent oxydant tel que le peroxyde d'hydrogène, les peracides et les persels.

On peut citer parmi les peracides et les persels, l'acide periodique et ses sels hydrosolubles, les permanganates et les bichromates, tels que de sodium ou de potassium, le persulfate d'ammonium et les peracides organiques.

Le sel d'acide periodique préféré est le periodate de sodium.

D'autres agents oxydants sont choisis parmi les chlorites alcalins, l'oxyde d'argent, le chlorure ferrique, l'oxyde de plomb, le nitrite de sodium; les sels de terres rares tels que notamment le sels de cérium.

On peut également utiliser des oxydants organiques choisis parmi les ortho et parabenzoquinones, les ortho et parabenzoquinones monoimines et diimines, les 1,2- et 1,4-naphtoquinones, les 1,2- et les 1,4-naphtoquinones mono- ou diimines.

L'oxydation peut enfin être effectuée par utilisation d'iodure tel qu'un iodure de métal alcalin, alcalino-terreux ou d'ammonium en présence de peroxyde d'hydrogène.

Ces agents oxydants peuvent être activés éventuellement par un agent modificateur de pH.

L'oxydation est généralement réalisée dans une gamme de températures allant de la température ambiante à 100°C avec une préférence pour les températures comprises entre 20 et 80°C.

II est également possible de procéder à la formation des produits indoliniques conformes a l'invention par oxydation par voie enzymatique. Cette oxydation s'effectue dans un milieu oxydant et d'une enzyme à activité oxydante ou peroxydante telle que les enzymes choisies parmi la peroxydase de raifort, la chloroperoxydase, la peroxydase du lait, la cytochrome C-peroxydase, ainsi que des produits ayant une activité similaire, celle des enzymes peroxydantes telle que l'hemoglobine, la methemoglobine, la myoglobine, la metmyoglobine. Cette oxydation enzymatique peut également s'effectuer en présence de tyrosinase avec l'oxygène de l'air.

Pour les produits destinés à une application cosmétique, on utilise de préférence comme agents oxydants, le peroxyde d'hydrogène, l'acide periodique et ses sels, le permanganate de potassium, l'hypochlorite de sodium, le persulfate d'ammonium, le nitrite de sodium et le système iodure/peroxyde d'hydrogène.

Lorsqu'on utilise un iodure en présence d'eau oxygénée, il s'agit préférentiellement d'iodure de sodium ou de potassium à une concentration pondérale comprise entre 1 et 6% par rapport au poids du milieu réactionnel.

L'ordre d'addition des composés intervenant dans la préparation du produit indolinique, conforme à l'invention, a peu d'importance à la condition que l'on incorpore en dernier lieu l'agent oxydant quand celui-ci est utilisé sans agent modificateur de pH et dans le cas du système oxydant iodure/peroxyde d'hydrogène, on introduit en dernier lieu, soit le peroxyde d'hydrogène, soit l'iodure.

Dans le cas où on utilise un agent modificateur de pH pour activer l'agent oxydant, on préfère ajouter en dernier lieu, soit l'agent oxydant, soit le modificateur de pH.

Les agents modificateurs de pH sont des agents acidifiants ou alcalinisants habituellement utilisés en cosmétique.

Avant l'addition de l'oxydant, le dérivé à oxyder est mis en solution aqueuse ou dans un milieu eau/solvant avec une proportion de solvant comprise entre 0,5 et 95% ou dans un milieu solvant pur.

Les solvants sont choisis parmi les alcools inférieurs en C₁-C₄, tels que l'alcool éthylique, l'alcool propylique ou isopropylique, l'alcool tertiobutylique, les alkylèneglycols tels que l'éthylèneglycol, le propyléneglycol, les alkyléthers d'alkylèneglycols tels que les éthers monométhylique, monoéthylique et monobutylique de l'éthylèneglycol, les monométhyléthers du propylèneglycol et du dipropylèneglycol et le lactate de méthyle.

Ces solvants doivent par ailleurs pouvoir solubiliser le composé indolinique et éventuellement le dérivé indolique mis en oeuvre pour former le produit indolinique.

Le solvant préféré est l'éthanol et le milieu préférentiel d'oxydation est hydroalcoolique avec une teneur en éthanol comprise entre 1 et 15%.

Dans le procédé conforme à l'invention, les dérivés à oxyder représentent en général de 0,1 à 30% et de préférence de 1 à 20% en poids du poids total du milieu réactionnel.

Les temps de contact entre les dérivés à oxyder et les réactifs oxydants peuvent varier de quelques minutes à quelques jours suivant les procédés.

Les agents alcalins préférés sont la soude, les carbonates alcalins ou l'ammoniaque. Lorsqu'ils sont utilisés, leur concentration dans le milieu d'oxydation est comprise entre 5.10⁻⁴ et 10% en poids.

Pour préparer les produits indoliniques conformes à l'invention, on utilise de préférence le procédé d'oxydation par l'eau oxygénée en présence d'ammoniaque.

Lorsque le processus d'oxydation est terminé, le produit indolinique coloré ainsi formé est isolé par filtration, centrifugation ou lyophilisation. Pour éliminer les traces de dérivés à oxyder n'ayant pas réagi, on rince le produit abondamment à l'eau avant ou après filtration ou centrifugation.

Dans le cas où l'on prépare le produit indolinique conforme à l'invention par simple oxydation à l'air, on préfère isoler le produit indolinique par lyophilisation.

Afin d'obtenir un produit homogène et de granulométrie suffisamment fine, il est ensuite possible de traiter le produit obtenu par des systèmes de broyages classiques par voie sèche ou humide. On peut également utiliser un procédé de micronisation.

La granulométrie du produit indolinique final doit être telle que le diamètre moyen des particules soit inférieur à 50 µm et de préférence inférieur à 20 µm. De même, 90% des particules ont un diamètre généralement inférieur à 100 microns et de préférence à 50 µm.

Les produits indoliniques conformes à l'invention, sont essentiellement des polymères généralement insolubles dans les milieux cosmétiques habituellement utilisés. Ces produits indoliniques peuvent être cependant mis en oeuvre en solution dans des milieux solvants particuliers, par exemple en utilisant un milieu dont le pH est élevé.

Les produits indoliniques conformes à l'invention selon un objet de l'invention, peuvent être utilisés en cosmétique, notamment dans tous les produits de traitement ou de soins de la peau et/ou des phanères. On appelle "phanères" les cheveux, les poils, cils, sourcils et les ongles.

Les produits indoliniques conformes à l'invention, dans leur application cosmétique, sont utilisés dans des compositions cosmétiques contenant dans un milieu cosmétiquement acceptable, à une concentration comprise de préférence entre 0,1 et 35% en poids et en particulier entre 0,5 et 20% en poids par rapport au poids total de la composition.

Ces compositions peuvent être utilisées comme produits de maquillage, notamment des cils, des sourcils, de la peau, des ongles, tels que sous forme de fards à paupières, fards à joues, ligneurs encore appelés "eye-liners", mascaras pour les cils et les sourcils, les vernis à ongles, comme compositions tinctoriales pour cheveux, notamment pour réaliser une teinture temporaire des cheveux ou maquillage.

Ces compositions peuvent également être utilisées pour la protection de l'épiderme humain contre les rayonnements UV.

Les compositions peuvent se présenter sous forme de lotion, de lotion épaissie, de gel, de crème, de lait, de poudre, de stick et éventuellement être conditionnées en aérosol et se présenter sous forme de mousse ou de spray.

Lorsque les compositions sont utilisées pour le maquillage de la peau, des cils et des sourcils, elles peuvent notamment se présenter sous forme solide ou pâteuse, anhydre ou aqueuse, comme des émulsions huile-dans-eau ou eau-dans-huile ou encore des suspensions.

Ces compositions présentent l'avantage d'être particulièrement stables et de présenter une bonne innocuité.

Lorsque les compositions sont utilisées pour la protection de l'épiderme humain contre les rayonnements UV, elles constituent des compositions dites "solaires" et elles peuvent se présenter sous forme de suspensions ou de dispersions dans des solvants ou des corps gras, ou encore sous forme d'émulsions telles que crèmes et laits, de pommades, de gels, de bâtonnets solides ou de mousses aérosols.

Lorsqu'elles sont utilisées sous forme d'émulsions, elles peuvent contenir en outre des agents tensio-actifs bien connus dans l'état de la technique, tels que des agents tensio-actifs anioniques, non-ioniques, cationiques ou amphotères.

Les compositions de maquillage et les compositions solaires peuvent également contenir des corps gras, des solvants organiques, des silicones, des épaississants, des adoucissants, des filtres solaires, des agents anti-mousses, des agents hydratants, des parfums, des conservateurs, des agents anti-oxydants, des charges, des séquestrants, des agents de traitement tels que des polymères anioniques, cationiques, non ioniques, amphotères, ou leurs mélanges, des propulseurs, des agents alcalinisants ou acidifiants.

Les corps gras peuvent être constitués par une huile ou une cire ou leur mélange, les acides gras, les alcools gras, la vaseline, la paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin.

Les cires sont choisies parmi les cires animales, fossiles, végétales, minérales ou de synthèse. On peut citer en particulier les cires d'abeilles, les cires de Carnauba, de Candellila, de canne à sucre, du Japon, les ozokérites, la cire de Montan, les cires microcristallines, les paraffines.

Les compositions conformes à l'invention peuvent également contenir en plus des produits indoliniques tels que définis ci-dessus, des pigments généralement utilisés en cosmétique, notamment des pigments nacrés et/ou nacrants permettant de varier les colorations susceptibles d'être obtenues, ou d'augmenter la protection vis-à-vis du rayonnement ultraviolet. On utilise, dans ce dernier cas, plus particulièrement des pigments ou nanopigments d'oxydes métalliques tels que les oxydes de titane, de zinc, de cérium ou de zirconium.

Les nanopigments qui sont utilisés de façon préférentielle, sont des pigments ayant un diamètre moyen inférieur à 100 nm et de préférence compris entre 5 et 50 nm. Ils peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans COSMETICS and TOILETRIES, Février 1990, Vol. 105, pages 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Lorsqu'elles sont utilisées pour la coloration temporaire des cheveux, elles se présentent sous forme de lotions plus ou moins épaissies, de gel, de mousse ou de spray contenant le produit indolinique dans un milieu aqueux ou eau/solvant(s) dans les proportions indiquées ci-dessus.

Lorsqu'elles sont utilisées pour le traitement des ongles, le produit indolinique est introduit dans un milieu pour vernis à ongles comprenant un solvant volatile et des polymères.

Un autre objet de l'invention est constitué par le procédé de coloration temporaire des cheveux, le maquillage de la peau et des phanères, de la protection de l'épiderme humain contre les effets néfastes des rayonnements UV, mettant en oeuvre les produits indoliniques selon l'invention.

Ces produits peuvent être appliqués directement sous forme de poudre ou au moyen de compositions cosmétiques telles que définies ci-dessus.

Les exemples suivants sont destinés à illustrer l'invention.

### EXEMPLES DE PREPARATION

### EXEMPLE 1

On solubilise 72,9 g (0,314 mole) de bromhydrate de 5,6-dihydroxyindoline dans 500 ml de solution aqueuse à 0,1% d'ammoniaque. On porte cette solution à 80°C et on ajoute une solution aqueuse de soude contenant 12,56 g (0,314 mole) de soude. On additionne à ce mélange 288 g d'eau oxygénée contenant 22,95 g (0,675 mole) de peroxyde d'hydrogène en maintenant la température entre 80 et 85° C. L'addition terminée, on maintient la température à 80°C pendant 2 heures puis on refroidit le milieu réactionnel. On essore le produit et on le lave à l'eau. On obtient après séchage 38,5 g de poudre noire. Cette poudre noire peut ensuite être micronisée selon les procédés classiques de micronisation.

Dans une variante de ce procédé le produit avant séchage est passé en milieu humide dans un broyeur à billes.

### EXEMPLE 2

On solubilise 43,8 g (0,188 mole) de bromhydrate de 5,6-dihydroxyindoline et 21,9 g (0,147 mole) de 5,6-dihydroxyindole dans 500 ml de solution aqueuse à 0,1 % d'ammoniaque. On porte ce mélange à 80 ° C et on ajoute une solution aqueuse de soude contenant 7,52 g (0,188 mole) de soude. On additionne à ce mélange en 2 h 30 minutes, 337,5 g d'eau oxygénée contenant 25,5 g (0,75 mole) de peroxyde d'hydrogène en maintenant la température entre 80° et 85°C. L'addition terminée, on maintient la température à 80°C pendant 3 heures puis on refroidit le milieu réactionnel. On essore le produit noir et on le lave à l'eau. On obtient après séchage 44,2 g de poudre noire qui peut, comme à l'exemple 1, être micronisée. Le produit noir obtenu avant séchage peut, comme à l'exemple 1, être passé en milieu humide dans un broyeur à billes.

### EXEMPLE 3

On dissout 0,12 g de tyrosinase de champignon dans 600 ml de tampon phosphate 0,2 M dont le pH est de 6,8 g. On ajoute 1,39 g (0,006 mole) de bromhydrate de 5,6-dihydroxyindoline sous agitation. On maintient pendant 4 heures l'agitation dans une atmosphère enrichie en oxygène à une température comprise entre 35° C et 37° C. On acidifie le milieu réactionnel à pH 3 avec de l'acide acétique. Le produit précipité est centrifugé, lavé avec 250 ml de solution à 0,1% d'acide acétique puis lavé à l'eau. Après lyophilisation, on obtient 0,93 g de poudre noire.

### EXEMPLE 4

On dissout 0,2 g de peroxydase de Raifort vendue par la Société SIGMA dans 4 litres de tampon acétate 0,2 M dont le pH est de 4,5. On ajoute 1 g (4,3.10⁻³ mole) de bromhydrate de 5,6-dihydroxyindoline sous agitation. On ajoute alors 8 ml d'eau oxygénée contenant 2,6 g (0,078 mole) de peroxyde d'hydrogène. On maintient l'agitation pendant 4 heures à une température comprise entre 35°C et 37°C. Le produit est isolé par centrifugation puis lavé à l'eau. Après lyophilisation, on obtient 0,62 g de poudre noire.

### EXEMPLE DE FORMULATION

### EXEMPLE 1

On prépare un mascara anhydre de composition suivante :

| | |
|---|---|
| - Cire de Carnauba | 5,0 g |
| - Cire de Candellila | 5,0 g |
| - Ethanol | 3,0 g |
| - Montmorillonite modifiée par une substance organique | 4,0 g |
| - Lanoline | 2,0 g |
| - Talc | 10,0 g |
| - Poudre noire de l'exemple 1 | 2,0 g |
| - Isoparaffine | qsp 100 g |

Ce mascara waterproof est de couleur noire.

### EXEMPLE 2

On prépare un mascara anhydre de composition suivante :

| | |
|---|---|
| - Cire de Carnauba | 5,0 g |
| - Cire de Candellila | 5,0 g |
| - Ethanol | 3,0 g |
| - Montmorillonite modifiée par une substance organique | 4,0 g |
| - Lanoline | 2,0 g |
| - Talc | 10,0 g |
| - Poudre noire de l'exemple 2 | 2,0 g |
| - Isoparaffine | qsp 100 g |

Ce mascara waterproof est de couleur noire.

Le mode opératoire est le suivant :

On chauffe les cires à 80°C. On ajoute le talc et les pigments. On incorpore ensuite la montmorillonite qui a été modifiée avec une substance organique et une partie de l'isoparaffine. A environ 40°C, on introduit l'alcool éthylique et le reste de l'isoparaffine. On passe le tout à la broyeuse.

Le mascara de l'exemple 1 est également préparé selon ce mode opératoire.

## Revendications

1. Produit indolinique, **caractérisé par le fait qu'**il est isolé sous forme de particules ayant une granulométrie moyenne inférieure à 50 µm et qu'il résulte de la polymérisation oxydative d'un ou plusieurs composés comprenant au moins un composé de formule (I) : 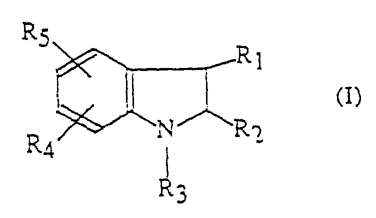 dans laquelle :
R₁ et R₃ représentent, indépendamment l'un de l'autre, un atome d'hydrogène ou un groupement alkyle en C₁-C₄;
R₂ représente un atome d'hydrogène, un groupement alkyle en C₁-C₄, un groupement carboxyle ou alcoxy(C₁-C₄)carbonyle;
R₄ désigne un atome d'hydrogène, un groupement alkyle en C₁-C₄, hydroxyle, alcoxy(C₁-C₄), amino ou alkylamino en C₁-C₁₀ ou halogène;
R₅ désigne un atome d'hydrogène, un groupement hydroxyle ou alcoxy en C₁-C₄ ou amino;
avec la condition qu'au moins un des radicaux R₄ ou R₅ désigne un groupement hydroxyle, alcoxy ou amino; et sous réserve que lorsque R₅ désigne un groupement amino, R₄ ne peut désigner un radical alkylamino;
R₄ et R₅ peuvent également former un cycle alkylènedioxy en C₁-C₂ et sont position 5 et 6;
ainsi que les sels correspondants.

2. Produit selon la revendication 1, **caractérisé par le fait que** les indolines de formule (I) sont choisies parmi la 5,6-dihydroxyindoline, la 6-hydroxyindoline, la 5,6-méthylènedioxyindoline, la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6-aminoindoline, la 5,6-diaminoindoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amine 6-chloroindoline. la 4-amino 6-iodoindoline, la 4-amino 5-bromoindoline, la 4-amino 5-hydroxyindoline, la 4-amino 7-hydroxyindoline, la 4-amino 5-méthoxyindoline, la 4-amino 7-méthoxyindoline, la 5-aminoindoline, la 2.3-diméthyl 5-aminoindoline, la 1-méthyl 5-aminoindoline, la 2-méthyl 5-aminoindoline, la 5-N-(1-méthylhexyl)aminoindoline, la 5,6-diméthexyindoline et la 5,6-dihydroxy 2-carboxyindoline.

3. Produit selon la revendication 1, **caractérisé par le fait qu'**il résulte de la cooxydation d'au moins un composé répondant à la formule (I) et d'un dérivé d'indole choisi parmi les mono- et dihydroxyindoles ou les aminoindoles dans des proportions de jusqu'à 50% en mole de dérivé indolique par rapport au nombre total de moles de composés à oxyder.

4. Produit selon la revendication 1, **caractérisé par le fait qu'**il résulte de la cooxydation d'au moins un composé de formule (I) et d'un dérivé d'indole de formule II 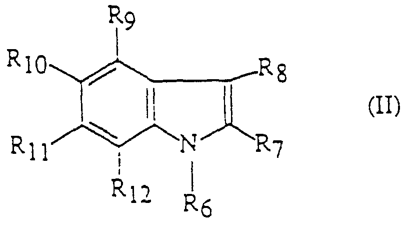 dans laquelle :
R₅ et R₈ désignent, indépendamment l'un de l'autre, un atome d'hydrogène ou un groupe alkyle en C₁-C₄;
R₇ représente un atome d'hydrogène, un groupement alkyle en C₁-C₄, un groupement carboxyle ou un groupement alcoxy(C₁-C₄) carbonyle;
R₉ et R₁₂ désignent, indépendamment l'un de l'autre, un atome d'hydrogène, un groupement hydroxy, un groupe alkyle en C₁-C₄, amino, alcoxy(C₁-C₄), acyl(C₂-C₄)oxy, acyl(C₂-C₄)amino;
R₁₀ désigne hydrogène, un groupement hydroxy, alcoxy(C₁-C₄), alkyle(C₁-C₄), halogène, amino, acyl(C₂-C₁₄)oxy, acyl(C₂-C₄)amino, triméthylsilyloxy;
R₁₁ désigne hydrogène un groupement hydroxy, alcoxy(C₁-C₄), amino, acyl(C₂-C₄)oxy, acyl(C₂-C₄)amino, triméthylsilyloxy, hydroxyalkyl(C₂-C₄)amino;
R₁₀ et R₁₁ peuvent former, conjointement avec les atomes de carbone auxquels ils sont rattachés, un cycle méthylènedioxy éventuellement substitué par un groupement alkyle en C₁-C₄ ou alcoxy en C₁-C₄ ou un cycle carbonyldioxy;
au moins l'un des groupements R₉ à R₁₂ représente un groupement OZ ou NHR, un seul au plus des groupements R₉ à R₁₂ désignant NHR;
et au plus deux des groupements R₉ à R₁₂ désignent OZ, dans le cas où Z désigne hydrogène, ces groupements sont en position 5 et 6;
et au moins un des groupements R₉ à R₁₂ représente hydrogène, dans le cas où un seul de ces groupements désigne hydrogène, un seul groupement parmi R₉ à R₁₂ désigne alors NHR ou OZ, et les autres groupements désignent alkyle en C₁-C₄;
R désignant dans NHR un atome d'hydrogène, un groupement acyle en C₂-C₄, hydroxyalkyle en C₂-C₄ et Z désignant dans OZ un atome d'hydrogène, un groupement acyle en C₂-C₁₄, alkyle en C₁-C₄, triméthylsilyle;
et les sels correspondants, dans des proportions de jusqu'à 50 % en moles de dérivé d'indole par rapport au nombre total de moles de composés à oxyder.

5. Procédé de préparation des produits selon l'une quelconque des revendications 1 à 4, **caractérisé par le fait que** l'on procède à l'oxydation à l'air en présence ou non d'un agent alcalin et/ou d'un catalyseur métallique d'oxydation, et qu'on isole le produit indolinique coloré ainsi formé par filtration, centrifugation ou lyophilisation.

6. Procédé de préparation des produits selon l'une quelconque des revendications 1 à 4, **caractérisé par le fait qu'**il est préparé par oxydation en présence d'un agent oxydant en présence ou non d'un agent modificateur de pH et/ou d'un catalyseur métallique d'oxydation suivie par l'isolation du produit indolinique coloré ainsi formé par filtration, centrifugation ou lyophilisation.

7. Procédé selon la revendication 6, **caractérisé par le fait que** l'agent oxydant est choisi parmi le peroxyde d'hydrogène, les peracides, les persels, les chlorites alcalins, l'oxyde d'argent, le chlorure ferrique, l'oxyde de plomb, le nitrite de sodium, les sels de terres rares.

8. Procédé selon la revendication 6, **caractérisé par le fait que** l'agent oxydant est choisi parmi les ortho- et les parabenzoquinones, les ortho- et parabenzoquinones monoimines ou diimines, les 1,2- et les 1,4-naphtoquinones, les 1,2- et les 1,4-naphtoquinones mono- ou diimines.

9. Procédé selon la revendication 7, **caractérisé par le fait que** l'oxydation est effectuée en utilisant dans un premier temps, soit un iodure d'un métal alcalin, alcalino-terreux ou d'ammonium, et dans un second temps, le peroxyde d'hydrogène, soit dans un premier temps le peroxyde d'hydrogène et suivi dans un second temps de l'addition d'un iodure de métal alcalin, alcalino-terreux ou d'ammonium.

10. Procédé de préparation des produits selon l'une quelconque des revendications 1 à 4, **caractérisé par le fait que** l'oxydation est effectuée par voie enzymatique suivie par l'isolation du produit indolinique coloré ainsi formé par filtration, centrifugation ou lyophilisation.

11. Procédé selon l'une quelconque des revendications 6 à 10, **caractérisé par le fait qu'**une oxydation est effectuée par introduction en solution aqueuse dans un milieu eau/solvant ou anhydre, du ou des indolines de formule (I) avec éventuellement des indoles, on additionne dans un second temps l'oxydant dans des quantités suffisantes pour former le produit indolinique.

12. Procédé selon la revendication 11, **caractérisé par le fait que** les solvants sont choisis parmi les alcools inférieurs en C₁-C₄, les alkylèneglycols, les alkyléthers d'alkylèneglycols, le lactate de méthyle.

13. Procédé selon l'une quelconque des revendications 5 à 12, **caractérisé par le fait que** les dérivés indoliniques et éventuellement les indoles, représentent de 0,1 à 30% en poids par rapport au poids du milieu réactionnel.

14. Produit selon l'une quelconque des revendications 1 à 4, **caractérisé par le fait qu'**il est isolé sous forme de particules ayant une granulométrie moyenne inférieure à 20 microns.

15. Utilisation du produit tel que défini dans l'une quelconque des revendications 1 à 4 et 14, en cosmétique.

16. Composition cosmétique, **caractérisée par le fait qu'**elle contient 0,1 à 35% en poids dans un milieu cosmétiquement acceptable, d'un produit tel que défini dans l'une quelconque des revendications 1 à 4 et 14.

17. Composition selon la revendication 16, **caractérisée par le fait qu'**elle se présente sous forme de lotion, de lotion épaissie, de gel, de crème, de lait, de poudre, de stick, qu'elle est éventuellement conditionnée en aérosol sous forme de spray ou de mousse.

18. Composition selon la revendication 16 ou 17, **caractérisée par le fait qu'**elle est destinée à être utilisée pour le maquillage de la peau, des ongles, des cils et des sourcils et qu'elle se présente sous forme liquide, solide ou pâteuse, anhydre ou aqueuse.

19. Composition selon l'une quelconque des revendications 16 à 18, **caractérisée par le fait qu'**elle est destinée à la protection de l'épiderme humain contre les rayonnements UV et qu'elle se présente sous forme de suspension ou de dispersion dans des solvants ou des corps gras ou sous forme d'émulsion, de pommade, de gel, de bâtonnet solide ou de mousse aérosol.

20. Composition selon l'une quelconque des revendications 16 à 18, **caractérisée par le fait qu'**elle contient des corps gras, des solvants organiques, des silicones, des épaississants, des adoucissants, des tensio-actifs. des filtres solaires, des agents anti-mousse, des agents hydratants, des parfums, des conservateurs, des agents anti-oxydants, des charges, des séquestrants, des agents de traitement, des propulseurs, des agents alcalinisants ou acidifiants ou d'autres pigments.

21. Procédé de coloration temporaire des cheveux, **caractérisé par le fait que** l'on utilise une composition telle que définie dans l'une quelconque des revendications 16, 17 et 20.

22. Procédé de maquillage de la peau ou des phanères, **caractérisé par le fait que** l'on applique sur la peau ou les phanères, une composition selon l'une quelconque des revendications 16 à 20.

## Patentansprüche

1. Indolin-Produkt,
**dadurch gekennzeichnet, dass** es in Form von Partikeln mit einer mittleren Korngröße unterhalb 50 µm isoliert wird und aus der oxidativen Polymerisation einer oder mehrerer Verbindungen resultiert, enthaltend mindestens eine Verbindung der Formel (I): 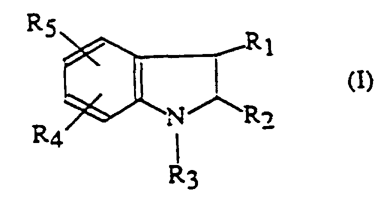 worin gilt:
R₁ und R₃ stellen, unabhängig voneinander, ein Wasserstoffatom oder eine C₁₋₄-Alkylgruppe dar;
R₂ stellt ein Wasserstoffatom, eine C₁₋₄-Alkyl-, Carboxyloder C₁₋₄-Alkoxycarbonylgruppe dar;
R₄ bedeutet ein Wasserstoffatom, eine C₁₋₄-Alkyl-, Hydroxyl-, C₁₋₄-Alkoxy-, Amino- oder C₁₋₁₀-Alkylaminogruppe oder ein Halogenatom;
R₅ bedeutet ein Wasserstoffatom, eine Hydroxyl-, C₁₋₄-Alkoxyoder Aminogruppe;
mit der Maßgabe, dass mindestens einer der Reste R₄ oder R₅ eine Hydroxyl-, Alkoxy- oder Aminogruppe bedeutet; und vorausgesetzt, dass, wenn R₅ eine Aminogruppe bedeutet, R₄ keinen Alkylaminorest bedeuten kann;
R₄ und R₅ können auch einen C₁-C₂-Alkylendioxy-Ring bilden und liegen dann in Position 5 und 6 vor; sowie die entsprechenden Salze.

2. Produkt gemäß Anspruch 1,
**dadurch gekennzeichnet, dass**
die Indoline der Formel (I) aus 5,6-Dihydroxyindolin, 6-Hydroxyindolin, 5,6-Methylendioxyindolin, 7-Methoxy-6-hydroxyindolin, 6,7-Dihydroxyindolin, 5-ydroxy-4-methoxyindolin, 4,5-Dihydroxyindolin, 5-Methoxy-6-hydroxyindolin, 4-Hydroxy-5-methoxyindolin, 5-Hydroxy-6-methoxyindolin, 4,7-Dihydroxyindolin, 6-Aminoindolin, N-Ethyl-4-hydroxyindolin, 1-Ethyl-6-aminoindolin, 5,6-Diaminoindolin, 1-Methyl-6-aminoindolin, 2-Methyl-6-aminoindolin, 3-Methyl-6-aminoindolin, 2-Methyl-5,6-diaminoindolin, 5-Chlor-7-aminoindolin, 3-Methyl-5,7-diaminoindolin, 5,7-Diaminoindolin, 2-Methyl-5,7-diaminoindolin, 7-Aminoindolin, 2-Methyl-7-aminoindolin, 4-Aminoindolin, 4-Amino-6-chlorindolin, 4-Amino-6-jodinollin, 4-Amino-5-bromindolin, 4-Amino-5-hydroxyindolin, 4-Amino-7-hydroxyindolin, 4-Amino-5-methoxyindolin, 4-Amino-7-methoxyindolin, 5-Aminoindolin, 2, 3-Dimethyl-5-aminoindolin, l-Methyl-5-aminoindolin, 2-Methyl-5-aminoindolin, 5-N-(1-Methylhexyl) aminoindolin, 5,6-Dimethoxyindolin sowie aus 5,6-Dihydroxy-2-carboxyindolin ausgewählt sind.

3. Produkt gemäß Anspruch 1,
**dadurch gekennzeichnet, dass**
es aus der Cooxidation mindestens einer Verbindung der Formel (I) und eines Indolderivats ausgewählt aus Mono- und Dihydroxyindolen oder aus Aminoindolen, in Mengenanteilen bis 50 Mol.% Indolderivat, bezogen auf die Gesamtzahl der Mole der zu oxidierenden Verbindungen, rsultiert.

4. Produkt gemäß Anspruch 3,
**dadurch gekennzeichnet, dass**
es aus der Cooxidation mindestens einer Verbindung der Formel (I) und eines Indolderivats der Formel (II): 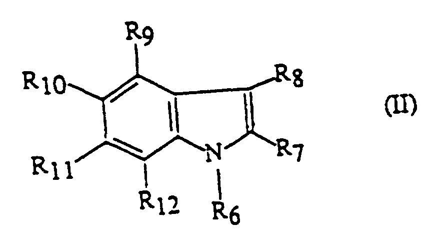 worin gilt:
R₆ und R₈ bedeuten, unabhängig voneinander, ein Wasserstoffatom oder eine C₁₋₄-Alkylgruppe;
R₇ stellt ein Wasserstoffatom, eine C₁₋₄-Alkyl-, Carboxyloder C₁₋₄-Alkoxycarbonalygruppe dar;
R₉ und R₁₂ bedeuten, unabhängig voneinander, ein Wasserstoffatom, eine Hydroxy-, C₁₋₄-Alkyl-, Amino-, C₁₋₄-Alkoxy-, C₂₋₄-Acyloxy- oder C₂₋₄-Acylaminogruppe;
R₁₀ bedeutet Wasserstoff, eine Hydroxy-, C₁₋₄-Alkoxy-, C₁₋₄-Alkyl-, Halogen-, Amino-, C₂₋₁₄-Acyloxy-, C₂₋₄-Acylyaminooder eine Trimethylsilyloxygruppe;
R₁₁ bedeutet Wasserstoff, eine Hydroxy-, C₁₋₄-Alkoxy-, Amino-, C₂₋₄-Acyloxy-, C₂₋₄-Acylamino-, Trimethylsilyloxioder eine Hydroxy-C₂₋₄-alkylaminogruppe;
R₁₀ und R₁₁ können, zusammen mit den Kohlenstoffatomen, an die sie gebunden sind, auch einen gegebenenfalls mit einer C₁₋₄-Alkyl- oder C₁₋₄-Alkoxygruppe substituierten Methylendioxy-Ring oder einen Carbonyldioxy-Ring bilden; mindestens eine der Gruppen R₉ bis R₁₂ stellt eine OZ- oder NHR-Gruppe dar, wobei höchstens eine einzige der Gruppen R₉ bis R₁₂ eine NHR-Gruppe darstellt;
und höchstens zwei der Gruppen R₉ bis R₁₂ bedeuten OZ, wobei, wenn Z Wasserstoff bedeutet, diese Gruppen in Position 5 und 6 vorliegen;
und mindestens eine der Gruppen R₉ bis R₁₂ stellt Wasserstoff dar, wobei, wenn eine einzige dieser Gruppen Wasserstoff bedeutet, eine einzige Gruppe unter R₉ bis R₁₀ dann eine NHRoder OZ-Gruppe und die weiteren Gruppen eine C₁₋₄-Alkylgruppe bedeuten,
wobei R in NHR ein Wasserstoffatom, eine C₂₋₄-Acyl- oder Hydroxy-C₂₋₄-alkylgruppe und Z in OZ ein Wasserstoffatom, eine C₂₋₁₄-Acyl-, C₁₋₄-Alkyl- oder eine Trimethylsilyloxigruppe bedeuten;
sowie der entsprechenden Salze in Mengenanteilen bis 50 Mol.-% Indolderivat, bezogen auf die Gesamtzahl der Mole der zu oxidierenden Verbindungen, resultiert.

5. Verfahren zur Herstellung von Produkten gemäß jedem der Ansprüche 1 bis 4,
**dadurch gekennzeichnet, dass**
man die Oxidation mit Luft in Gegenwart oder Abwesenheit eines alkalischen Mittels und/oder eines metallhaltigen Oxidationskatalysators durchführt und das so gebildete gefärbte Indolin-Produkt durch Filtrieren, Zentrifugieren oder Lyophilisieren isoliert.

6. Verfahren zur Herstellung von Produkten gemäß jedem der Ansprüche 1 b 4,
**dadurch gekennzeichnet, dass**
sie durch Oxidation in Gegenwart eines oxidierenden Mittels in der Gegenwart oder Abwesenheit eines pH-Modifiziermittels und/oder eines metallhaltigen Oxidationskatalysators hergestellt werden, worauf das so gebildete gefärbte Indolinprodukt durch Filtrieren, Zentrifugieren und Lyophilisieren isoliert wird.

7. Verfahren gemäß Anspruch 6,
**dadurch gekennzeichnet, dass**
das oxidierende Mittel aus Wasserstoffperoxid, Persäuren, Persalzen, Alkalichloriten, Silberoxid, Ferrichlorid, Bleioxid, Natriumnitrit und aus Salzen von seltenen Erden ausgewählt ist.

8. Verfahren gemäß Anspruch 6,
**dadurch gekennzeichnet, dass**
das oxidierende Mittel aus o- und p-Benzochinonen, o- und p-Benzochinonmonoiminen und -diiminen, 1,2- und 1,4-Naphthochinonen, 1,2- und 1,4-Naphthochinonmono- oder -diiminen ausgewählt ist.

9. Verfahren gemäß Anspruch 7,
**dadurch gekennzeichnet, dass**
die Oxidation durchgeführt wird, indem man zuerst entweder ein Alkalimetall-, Erdalkali- oder Ammoniumjodid und zu einem zweiten Zeitpunkt das Wasserstoffperoxid oder indem man zuerst das Wasserstoffperoxid und dann zu einem zweiten Zeitpunkt ein Alkali-, Erdalkali- oder Ammoniumjodid zufügt.

10. Verfahren zur Herstellung von Produkten gemäß jedem der Ansprüche 1 bis 4,
**dadurch gekennzeichnet, dass**
die Oxidation auf enzymatischem Weg durchgeführt wird, worauf das so gebildete gefärbte Indolin-Produkt durch Filtrieren, Zentrifugieren und lyophilisieren isoleirt wird.

11. Verfahren gemäß jedem der Ansprüche 6 bis 10,
**dadurch gekennzeichnet, dass**
eine Oxidation durch Einbringung in wässrige Lösung in einem Milieu aus Wasser/Lösungsmittel oder in ein wasserfreies Milieu des oder der Indoline der Formel (I) mit gegebenenfalls Indolen durchgeführt wird und man zu einem zweiten Zeitpunkt das Oxidationsmittel in zur Bildung des Indolin-Produkts ausreichenden Mengen zufügt.

12. Verfahren gemäß Anspruch 11,
**dadurch gekennzeichnet, dass**
die Lösungsmittel aus C₁₋₄-Niedrigalkoholen, Alkylenglycolen, Alkylethern von Alkylenglycolen und aus Methyllactat ausgewählt sind.

13. Verfahren gemäß jedem der Ansprüche 5 bis 12,
**dadurch gekennzeichnet, dass**
die Indolin-Derivate und gegebenenfalls die Indole 0,1 bis 30 Gew.% ausmachen, bezogen auf das Gesamtgewicht des Reaktionsmediums.

14. Produkt gemäß jedem der Ansprüche 1 bis 4,
**dadurch gekennzeichnet, dass**
es in Form von Partikeln mit einer mittleren Korngröße von weniger als 20 µm gewonnen wird.

15. Verwendung des Produkts gemäß jedem der Ansprüche 1 bis 4 und 14 in der Kosmetik.

16. Kosmetische Zusammensetzung,
**dadurch gekennzeichnet, dass**
sie in einem kosmetisch geeigneten Milieu 0,1 bis 35 Gew.% eines Produkts gemäß jedem der Ansprüche 1 bis 4 und 14 enthält.

17. Zusammensetzung gemäß Anspruch 16,
**dadurch gekennzeichnet, dass**
sie in Form einer Lotion, verdickten Lotion, eines Gels, einer Creme, einer Milch, eines Pulvers, eines Stifts vorliegt und gegebenenfalls als Aerosol in Form eines Spray-Produkts oder Schaums zubereitet ist.

18. Zusammensetzung gemäß Anspruch 16 oder 17,
**dadurch gekennzeichnet, dass**
sie zur Verwendung zum Schminken der Haut, der Nägel, der Augenbrauen und der Wimpern bestimmt ist und in flüssiger, fester oder pastenartiger, wasserfreier oder wässriger Form vorliegt.

19. Zusammensetzung gemäß jedem der Ansprüche 16 bis 18,
**dadurch gekennzeichnet, dass**
sie zum Schutz der menschlichen Epidermis vor den UV-Strahlungen bestimmt ist und in Form einer Suspension oder Dispersion in Lösungsmitteln oder Fettkörpern oder in Form einer Emulsion, Pomade, eines Gels, Feststoffstäbchens oder Aerosol-Schaums vorliegt.

20. Zusammensetzung gemäß jedem der Ansprüche 16 bis 18,
**dadurch gekennzeichnet, dass**
sie Fettkörper, organische Lösungsmittel, Silikone, Verdickungsmittel, Weichmacher, oberflächenaktive Mittel, Sonnenfiltermittel, Antischaummittel, hydratisierende Mittel, Parfüm-Produkte, Konservierungsstoffe, Antioxidantien, Beaufschlagungsmittel, Sequestriermittel, Behandlungsmittel, Treibmittel, alkalisch oder sauer stellende Mittel oder weitere Pigmente enthält.

21. Verfahren zur kurzzeitigen Färbung der Haare,
**dadurch gekennzeichnet, dass**
man eine Zusammensetzung gemäß einem der Ansprüche 16, 17 und 20 verwendet.

22. Verfahren zum Schminken der Haut oder von Sichtstellen,
**dadurch gekennzeichnet, dass**
man auf die Haut oder die Sichtstellen eine Zusammensetzung gemäß jedem der Ansprüche 16 bis 20 aufbringt.

## Claims

1. Indoline-based product, **characterized in that** it is isolated in the form of particles having an average particle size of less than 50 µm and **in that** it results from the oxidative polymerization of one or more compounds comprising at least one compound of formula (I): 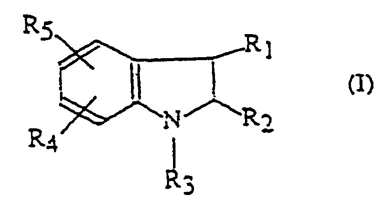 in which:
R₁ and R₃ represent, independently of one another, a hydrogen atom or a C₁-C₄ alkyl group;
R₂ represents a hydrogen atom, a C₁-C₄ alkyl group or a carboxyl or (C₁-C₄ alkoxy)carbonyl group;
R₄ denotes a hydrogen atom or a C₁-C₄ alkyl, hydroxyl, (C₁-C₄ alkoxy), amino or C₁-C₁₀ alkylamino or halogen group;
R₅ denotes a hydrogen atom or a hydroxyl, C₁-C₄ alkoxy or amino group;
on condition that at least one of the radicals R₄ or R₅ denotes a hydroxyl, alkoxy or amino group, and with the proviso that when R₅ denotes an amino group, R₄ cannot denote an alkylamino radical;
R₄ and R₅ can also form a C₁-C₂ alkylenedioxy ring, and are at positions 5 and 6;
as well as the corresponding salts.

2. Product according to Claim 1, **characterized in that** the indolines of formula (I) are chosen from 5,6-dihydroxyindoline, 6-hydroxyindoline, 5,6-methylenedioxyindoline, 7-methoxy-6-hydroxyindoline, 6,7-dihydroxyindoline, 5-hydroxy-4-methoxyindoline, 4,5-dihydroxyindoline, 5-methoxy-6-hydroxyindoline, 4-hydroxy-5-methoxyindoline, 5-hydroxy-6-methoxyindoline, 4,7-dihydroxyindoline, 6-aminoindoline, N-ethyl-4-hydroxyindoline, 1-ethyl-6-aminoindoline, 5,6-diaminoindoline, 1-methyl-6-aminoindoline, 2-methyl-6-aminoindoline, 3-methyl-6-aminoindoline, 2-methyl-5,6-diaminoindoline, 5-chloro-7-aminoindoline, 3-methyl-5,7-diaminoindoline, 5,7-diaminoindoline, 2-methyl-5,7-diaminoindoline, 7-aminoindoline, 2-methyl-7-aminoindoline, 4-aminoindoline, 4-amino-6-chloroindoline, 4-amino-6-iodoindoline, 4-amino-5-bromoindoline, 4-amino-5-hydroxyindoline, 4-amino-7-hydroxyindoline, 4-amino-5-methoxyindoline, 4-amino-7-methoxyindoline, 5-aminoindoline, 2,3-dimethyl-5-aminoindoline, 1-methyl-5-aminoindoline, 2-methyl-5-aminoindoline, 5-[N-(1-methylhexyl)amino]-indoline, 5,6-dimethoxyindoline and 5,6-dihydroxy-2-carboxyindoline.

3. Product according to Claim 1, **characterized in that** it results from the cooxidation of at least one compound corresponding to the formula (I) and an indole derivative chosen from mono- and dihydroxyindoles or aminoindoles, in proportions of up to 50 mol% of indole derivative relative to the total number of moles of compounds to be oxidized.

4. Product according to Claim 1, **characterized in that** it results from the cooxidation of at least one compound of formula (I) and an indole derivative of formula II 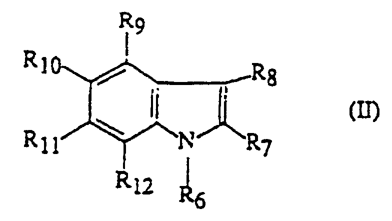 in which:
R₆ and R₈ denote, independently of one another, a hydrogen atom or a C₁-C₄ alkyl group;
R₇ represents a hydrogen atom, a C₁-C₄ alkyl group, a carboxyl group or a (C₁-C₄ alkoxy) carbonyl group;
R₉ and R₁₂ denote, independently of one another, a hydrogen atom, a hydroxyl group or a C₁-C₄ alkyl, amino, (C₁-C₄ alkoxy), (C₂-C₄ acyl)oxy or (C₂-C₄ acyl)amino group;
R₁₀ denotes hydrogen or a hydroxyl, (C₁-C₄ alkoxy), (C₁-C₄ alkyl), halogen, amino, (C₂-C₁₄ acyl)oxy, (C₂-C₄ acyl)amino or trimethylsilyloxy group;
R₁₁ denotes hydrogen or a hydroxyl, (C₁-C₄ alkoxy), amino, (C₂-C₄ acyl)oxy, (C₂-C₄ acyl)amino, trimethylsilyloxy or hydroxy(C₂-C₄ alkyl)amino group;
R₁₀ and R₁₁, together with the carbon atoms to which they are attached, can form a methylenedioxy ring optionally substituted with a C₁-C₄ alkyl or C₁-C₄ alkoxy group or a carbonyldioxy ring;
at least one of the groups R₉ to R₁₂ represents a group OZ or NHR, not more than one of the groups R₉ to R₁₂ denoting NHR;
and not more than two of the groups R₉ to R₁₂ denote OZ, in the case where Z denotes hydrogen, these groups are at positions 5 and 6;
and at least one of the groups R₉ to R₁₂ represents hydrogen, in the case where only one of these groups denotes hydrogen, only one group from among R₉ to R₁₂ then denotes NHR or OZ and the other groups denote C₁-C₄ alkyl;
R in NHR denoting a hydrogen atom or a C₂-C₄ acyl or C₂-C₄ hydroxyalkyl group, and Z in OZ denoting a hydrogen atom or a C₂-C₁₄ acyl, C₁-C₄ alkyl or trimethylsilyl group; and the corresponding salts, in proportions of up to 50 mol% of indole derivative relative to the total number of moles of compounds to be oxidized.

5. Process for preparing the products according to any one of Claims 1 to 4, **characterized in that** oxidation is carried out in air in the presence or absence of an alkaline agent and/or of a metal-based oxidation catalyst, and **in that** the coloured indoline-based product thereby formed is isolated by filtration, centrifugation or lyophilization.

6. Process for preparing the products according to any one of Claims 1 to 4, **characterized in that** they are prepared by oxidation in the presence of an oxidizing agent, in the presence or absence of a pH-modifying agent and/or of a metal-based oxidation catalyst, followed by isolation of the coloured indoline-based product thereby formed by filtration, centrifugation or lyophilization.

7. Process according to Claim 6, **characterized in that** the oxidizing agent is chosen from hydrogen peroxide, peracids, persalts, alkali metal chlorites, silver oxide, ferric chloride, lead oxide, sodium nitrite and rare-earth salts.

8. Process according to Claim 6, **characterized in that** the oxidizing agent is chosen from ortho- and para-benzoquinones, ortho- and para-benzoquinone monoimines or diimines, 1,2- and 1,4-naphthoquinones and 1,2- and 1,4-naphthoquinone mono- or diimines.

9. Process according to Claim 7, **characterized in that** the oxidation is performed using, in a first stage, either an alkali metal iodide, alkaline-earth metal iodide or ammonium iodide, and, in a second stage, hydrogen peroxide, or, in a first stage, hydrogen peroxide and followed in a second stage by the addition of an alkali metal iodide, alkaline-earth metal iodide or ammonium iodide.

10. Process for preparing the products according to any one of Claims 1 to 4, **characterized in that** the oxidation is performed enzymatically, followed by isolation of the coloured indoline-based product thereby formed by filtration, centrifugation or lyophilization.

11. Process according to any one of Claims 6 to 10, **characterized in that** an oxidation is performed by introduction, in aqueous solution in a water/solvent or anhydrous medium, of the indoline or indolines of formula (I) with, where appropriate, indoles, and, in a second stage, the oxidizing agent is added in sufficient amounts to form the indoline-based product.

12. Process according to Claim 11, **characterized in that** the solvents are chosen from C₁-C₄ lower alcohols, alkylene glycols, alkylene glycol alkyl ethers and methyl lactate.

13. Process according to any one of Claims 5 to 12, **characterized in that** the indoline derivatives and, where appropriate, the indoles represent from 0.1 to 30% by weight relative to the weight of the reaction medium.

14. Product according to any one of Claims 1 to 4, **characterized in that** it is isolated in the form of particles having an average particle size of less than 20 microns.

15. Use of the product as defined in any one of Claims 1 to 4 and 14, in cosmetics.

16. Cosmetic composition, **characterized in that** it contains 0.1 to 35% by weight, in a cosmetically acceptable medium, of a product as defined in any one of Claims 1 to 4 and 14.

17. Composition according to Claim 16, **characterized in that** it takes the form of a lotion, thickened lotion, gel, cream, milk, powder or stick, and **in that** it is optionally packaged as an aerosol in the form of a spray or foam.

18. Composition according to Claim 16 or 17, **characterized in that** it is intended for use for making up the skin, nails, eyelashes and eyebrows, and **in that** it takes anhydrous or aqueous pasty, solid or liquid form.

19. Composition according to any one of Claims 16 to 18, **characterized in that** it is intended for protection of the human epidermis against UV radiation, and **in that** it takes the form of a suspension or dispersion in solvents or fatty substances or the form of an emulsion, pomade, gel, solid stick or aerosol foam.

20. Composition according to any one of Claims 16 to 18, **characterized in that** it contains fatty substances, organic solvents, silicones, thickeners, demulcents, surfactants, sunscreens, antifoams, hydrating agents, fragrances, preserving agents, antioxidants, fillers, sequestering agents, treatment agents, propellants, basifying or acidifying agents or other pigments.

21. Process for the temporary dyeing of hair, **characterized in that** a composition as defined in any one of Claims 16, 17 and 20 is used.

22. Process for making up the skin or the integuments, **characterized in that** a composition according to any one of Claims 16 to 20 is applied to the skin or the integuments.
